(19) Europäisches Patentamt
European Patent Office
Office européen des brevets

(11) Publication number: **0 146 370**
**B1**

(12) # EUROPEAN PATENT SPECIFICATION

(45) Date of publication of patent specification: **19.10.88**

(21) Application number: **84308764.4**

(22) Date of filing: **14.12.84**

(51) Int. Cl.⁴: **C 07 D 471/06,** A 61 K 31/47, C 07 D 487/06 // (C07D471/06, 235:00, 221:00)

(54) **Imidazoquinolines containing other heterocyclic groups.**

(30) Priority: **16.12.83 GB 8333580**
**28.09.84 GB 8424607**

(43) Date of publication of application:
**26.06.85 Bulletin 85/26**

(45) Publication of the grant of the patent:
**19.10.88 Bulletin 88/42**

(84) Designated Contracting States:
**AT BE CH DE FR IT LI LU NL SE**

(56) References cited:
**GB-A-2 039 218**
**GB-A-2 069 492**
**US-A-3 200 123**
**US-A-4 409 226**

(73) Proprietor: **JOHN WYETH & BROTHER LIMITED**
**Huntercombe Lane South Taplow**
**Maidenhead Berkshire, SL6 0PH (GB)**

(72) Inventor: **Crossley, Roger**
**45 Hazel Drive**
**Woodley Reading Berkshire (GB)**

(74) Representative: **Porter, Graham Ronald et al**
**c/o John Wyeth & Brother Limited**
**Huntercombe Lane South Taplow**
**Maidenhead Berkshire, SL6 0PH (GB)**

**EP 0 146 370 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to novel imidazoquinoline derivatives useful for the treatment of ulcers or hypersecretion in mammals, to processes for their preparation and to pharmaceutical compositions containing the novel compounds.

In our GB Patent Specification 2069492A we have described *inter alia* phenylsulphinylalkylpyridines which are useful in the treatment of ulcers or hypersecretion in mammals. We have now found that by modifying the molecule to replace the phenyl group by an imidazoquinoline group novel compounds are obtained which have one or more of the following activities, anti-ulcer, anti-secretory or $H^+/K^+$ ATPase inhibitory activity. The pyridyl group can also be replaced by other Heterocyclic groups as detailed below.

US Patent 3200123 discloses 5,6-dihydroimidzo[ij] quinoline derivatives of Formula A

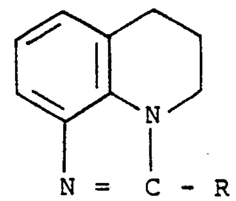

A

where R may be a halogen, mercapto, mercaptoalkyl, hydroxy, trihalomethyl, amino, carboxy, dialkylaminoalkylamino, alkoxy, aryl, heterocyclic or heterocyclic substituted alkyl radical of various kinds. The compounds are said to be anti-inflammatory agents and some of the compounds have uterotrophic activity, hypnotic or analgesic properties.

GB Patent 1,525,958 discloses 2-[heterocyclic substituted alkylsulphinyl]-benzimidazoles which have anti-secretory activity.

The invention provides a compound of formula I

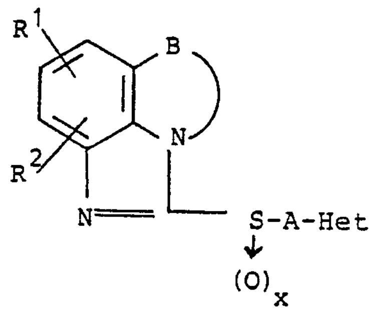

(I)

wherein A is a $C_1$—$C_4$ straight or branched alkylene chain which may be saturated or unsaturated, B is a $C_2$—$C_4$ straight or branched alkylene chain which may be saturated or unsaturated, $R^1$ and $R^2$ are the same or different and are hydrogen $C_1$—$C_6$-alkyl, $C_1$—$C_6$-alkoxy, $C_1$—$C_6$-alkoxyl($C_1$—$C_6$)alkyl, $C_1$—$C_6$ hydroxyalkyl, hydroxy, halogen, nitro, carboxy, carboxylic ester, carbamoyl, carbamoyloxy, $C_2$—$C_7$ alkanoylamino such as acetamino, cyano, $C_2$—$C_7$ alkanoyl or trifluoromethyl, Het is a heterocyclic group chosen from imidazolyl, imidazolinyl, benzimidazolyl, thiazolyl, thiazolinyl, quinolyl, piperidyl, pyridyl, benzothiazolyl and pyrimidyl, any of which heterocyclic groups may be substituted as described below and x is 0 or 1, and pharmaceutically acceptable salts thereof.

Examples of A are $CH_2$, $CH(CH_3)$, $CH(CH_3)CH_2$, $CH_2CH_2$, $CH_2CH_2CH_2$, $CH{=}CH$, and $CH{=}CHCH_2$. Preferably A is $CH_2$.

B may be for example $CH_2CH_2$, $CH(CH_3)CH_2$, $CH_2CH_2CH_2$, $CH{=}CH$, $CH{=}CHCH_2$ or $(CH_2)_4$.

The group Het may be mono or polysubstituted but is preferably mono or disubstituted by any of the following: halogen, $C_1$—$C_6$ alkoxy, $C_7$—$C_{12}$ aralkoxy, hydroxy, $C_1$—$C_6$ hydroxyalkyl, $C_1$—$C_6$ alkoxy($C_1$—$C_6$alkyl, trifluoromethyl, $C_1$—$C_6$ alkyl, phenyl or aralkyl of 7 to 12 carbon atoms or disubstituted by a loweralkylene dioxy radical of 1 to 6 carbon atoms. The group Het is preferably joined at the 2-position but may be joined at other positions eg 4-thiazoyl.

In this specification an alkyl group is preferably lower alkyl i.e. of 1 to 6 carbon atoms eg methyl, ethyl, n-propyl, isopropyl, n-butyl, s-butyl, t-butyl, n-pentyl or n-hexyl. An alkoxy group is preferably lower alkoxy in which the alkyl portion is as defined for a lower alkyl group. Whenever the term lower alkyl or lower alkoxy is used as part of another group e.g. arylloweralkyl, the lower alkyl or lower alkoxy portion has 1 to 6 carbon atoms unless otherwise stated. An aralkyl radical is preferably arylloweralkyl eg benzyl, phenethyl or phenpropyl. A phenyl group or aryl portion of another group such as aralkyl may be substituted e.g. by any of the substituents mentioned for the group Het.

Preferably an aralkyl group is phenylalkyl of 7—12 carbon atoms. A particularly preferred group of compounds within the scope of formula I are those in which Het is pyridyl e.g. 2-pyridyl, which may be substituted by any of the substituents described for Het, but phenyl or substituted phenyl are particularly preferred substituents especially when x is 0. Lower alkyl substituents are also of interest. These

compounds have especially interesting anti-secretory activity as measured by their ability to inhibit the highly specific proton transporting enzyme $H^+/K^+$ ATPase.

Preferred groups of compounds are those of formula I where $R^1$ and $R^2$ are selected from hydrogen $C_1$—$C_6$ alkyl and $C_1$—$C_6$ alkoxy and those where Het is 2-(6-phenylpyridyl).

A particularly preferred group of compounds may be represented by formula (Ia)

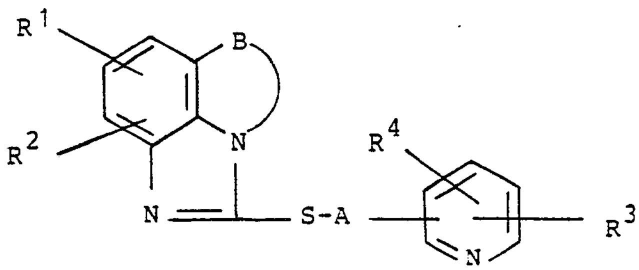

(Ia)

wherein A, $R^1$ and $R^2$ are as defined in connection with formula I above, $R^3$ and $R^4$ are the same or different and are hydrogen, lower alkyl or phenyl which may be substituted by any of the substituents described for Het, and one of $R^3$ and $R^4$ is other than hydrogen, and pharmaceutically acceptable salts thereof. The pharmaceutically acceptable salts are preferably acid addition salts.

The acid addition salts of compounds of formula I or Ia may be of any pharmaceutically acceptable organic or inorganic acid e.g. hydrochloric, hydrobromic, hydroiodic, phosphoric, sulphuric, nitric, citric, acetic, formic, fumaric, maleic, tartaric, embonic, malonic, alkylsulphonic, e.g. methanesulphonic, arylsulphonic, e.g. p-toluene sulphonic acids.

The invention includes methods of preparing the compounds of formula I.

The compounds of formula I, wherein x is 1, i.e. the sulphinyl compounds may be prepared by oxidising a corresponding compound of formula I where x is 0, or a pharmaceutically acceptable salt thereof, and if desired converting the product to an acid addition salt.

Oxidation may be effected by any suitable means for forming a sulphoxide from a thioether (see Kharasch, Organic Sulphur Compounds, Pergamon Press, New York, 1961, Volume 1, pages 157—159), for example by a per acid or peroxide. Examples of per acids are perbenzoic, m-chloroperbenzoic or peracetic acid. Hydrogen peroxide may also be used. Preferably an acid addition salt of compound II is used in order to prevent or minimise attack of the pyridine nitrogen by the oxidising agent.

The compounds of formula I, where x is 0, may be prepared by reacting a compound of formula II,

Het-A-Hal (II)

wherein Het and A are as defined above and Hal is a halogen atom especially chlorine, bromine or iodine, with a thiol of formula III, or an alkali metal salt thereof,

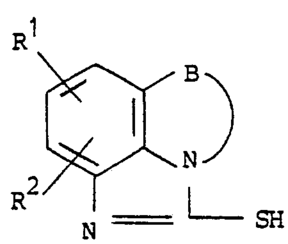

(III)

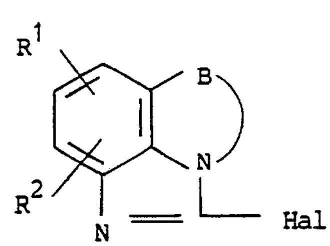

(IIIa)

wherein B, $R^1$ and $R^2$ are as defined above.

Alternatively a compound of formula IIa Het-A-SH, or an alkali metal salt thereof, may be reacted with a halide of formula IIIa wherein $R^1$, $R^2$, B and Hal are as defined above.

The starting compounds of formula II and IIa are known compounds or may be prepared by methods known for analogous compounds. The starting compounds of formula III or IIIa are known compounds described in for example J. Org. Chem. 1960, *25,* 1138 or 1963, *28,* 2581 or may be prepared by methods known for analogous compounds. In the above mentioned reaction the compounds of formula I may be isolated in free base form or as acid addition salts.

Further methods for preparing compounds of formula I include:

reacting a compound of formula IV

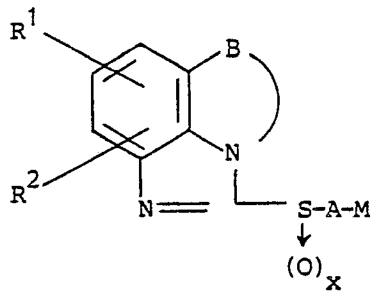

(IV)

wherein $R^1$, $R^2$, A, B and x are as defined above and M is sodium, potassium or lithium with a compound of formula

Het-Q (V)

where Het is as defined above and Q is a leaving group such as a reactive esterified hydroxy group or halogen.

The compounds of formula IV and V are known compounds or may be prepared by methods known for analogous compounds.

The reactive esterified hydroxy group is preferably an hydroxy group esterified with an organic sulphonic acid eg. benzene sulphonic acid or p-toluene sulphonic acid.

The compounds of formula I and their pharmaceutically acceptable salts, possess anti-ulcer and/or anti-secretory activity as measured by standard test procedures and accordingly are useful for the treatment of ulcers or hypersecretion in mammals. The compounds of formula I where x is 0 and their pharmaceutically acceptable salts are also intermediates for the corresponding compounds where x is 1.

Anti-ulcer activity was determined by the stress-induced erosion test of Senay and Levine, Proc. Soc. Exp. Biol. Med., *124,* 1221—3 (1967).

Anti-secretory activity was demonstrated by the test of H. Shay, D. Sun and H. Gruenstein, Gastroenterology, 1954, *26,* 903—13 as exemplified by Beattie *et al,* J. Med. Chem. 20, 714 (1977).

Compounds of formula I were also tested for anti-secretory activity by their ability to inhibit the highly specific proton transporting enzyme $H^+/K^+$ ATPase.

Potential $H^+/K^+$ ATPase inhibitors are evaluated by a technique involving the measurement of aminopyrine accumulation in rabbit isolated gastric glands. Aminopyrine, which is a weak base, accumulates in acid-secreting cells; therefore, up take of aminopyrine is increased by secretagogues and an inhibitor of acid secretion will reduce the response to one or more secretagogues depending upon its site of action. Compounds which reduce the response to dibutyryl cyclic adenosine monophosphate (DBcAMP) stimulation are assumed to have an intracellular site of action, and those which reduce the response to both DBcAMP and high potassium ion concentration ($K^+$) are thought to have an intracellular site of action at the secretory surface of the parietal cell, involving the highly specific proton — transporting enzyme, $H^+/K^+$ ATPase. The following test procedure is used:

Rabbit gastric glands are isolated from gastric mucosa from the corpus region of the stomach by a method based on one described by Berglindh T., Obrink K. J. Acta Physiol. Scand. *96,* 150—159 (1976). Measurement of aminopyrine uptake is carried out using a modification of the method described by Berglindh T., Hellander H. F., Obrink K. J. (ibid, *97,* 401—414, 1976).

Compounds are tested at a concentration of $10^{-4}$M, initially, and in some cases at lower concentrations, for their ability to inhibit $^{14}$C-aminopyrine uptake in gastric glands, stimulated by DBcAMP and high $K^+$ respectively. Results are expressed as the % inhibition of the maximum response to the secretagogue induced by the test compound. An inhibitor of $H^+/K^+$ ATPase would be expected to reduce the response to both secretagogues.

The invention also provides pharmaceutical compositions comprising a compound of formula I as defined above wherein x is 0 or 1, or a pharmaceutically acceptable salt thereof and a pharmaceutically acceptable carrier.

The pharmaceutical formulations include solids and liquids. Any suitable carrier known in the art can be used to prepare the pharmaceutical composition. In such a composition, the carrier is generally a solid or liquid, or a mixture of a solid and a liquid.

Solid form compositions include powders, granules, tablets and capsules (eg. hard and soft gelatin capsules). A solid carrier can be, for example, one or more substances which may also act as flavouring agents, lubricants, solubilisers, suspending agents, fillers, glidants, compression aids, binders, effervescent excipients or tablet-disintegrating agents; it can also be an encapsulating material. In powders the carrier is a finely divided solid which is in admixture with the finely divided active ingredient. In tablets the active ingredient is mixed with a carrier having the necessary compression properties in suitable proportions and compacted in the shape and size desired. The powders and tablets preferably contain up to 99%, eg. from 10 to 80%, preferably 25 to 75% of the active ingredient. Suitable solid carriers include, for

example, calcium phosphate, magnesium stearate, talc, sugars, lactose, dextrine, starch, gelatin, cellulose, methyl cellulose, sodium carboxymethyl cellulose, polyvinylpyrrolidone, low melting waxes and ion exchange resins.

The term "composition" is intended to include the formulation of an active ingredient with encapsulating material as carrier to give a capsule in which the active ingredient (with or without other carriers) is surrounded by the carrier, which is thus in association with it. Similarly cachets are included.

Liquid form compositions include, for example, suspensions, emulsions, syrups and elixirs. The active ingredient, for example, can be suspended in a pharmaceutically acceptable liquid carrier such as water, an organic solvent, a mixture of both or pharmaceutically acceptable oils or fats. The liquid carrier can contain other suitable pharmaceutical additives such as solubilizers, emulsifiers, buffers, preservatives, sweeteners, flavouring agents, suspending agents, thickening agents, colours, viscosity regulators, stabilisers or osmo-regulators. Suitable examples of liquid carriers for oral administration include water (particularly containing additives as above eg. cellulose derivatives, preferably sodium carboxymethyl cellulose solution) alcohols (including monohydric alcohols and polyhydric alcohols eg. glycerol and glycols) and their derivatives, and oils (eg. fractionated coconut oil and arachis oil).

Preferably the pharmaceutical composition is in unit dosage form, eg. as tablets or capsules. In such form, the composition is sub-divided in unit dose containing appropriate quantities of the active ingredient; the unit dosage forms can be packaged compositions, for example, packeted powders. The unit dosage form can be, for example, a capsule or tablet itself, or it can be the appropriate number of any such compositions in package form. The quantity of the active ingredient in a unit dose of composition may be varied or adjusted from 10 mg or less to 50 mg or more, according to the particular need.

The anti-ulcer composition of the invention will be administered orally on either liquid or solid composition form. These compositions may include one or more antacid ingredients, eg. aluminium hydroxide, magnesium hydroxide or bismuth carbonate, aluminium glycinate, calcium carbonate, magnesium trisilicate, sodium bicarbonate or the alumina gel described in British Specification No. 1,284,394.

In another aspect the invention provides as pharmaceutical, eg. an anti-ulcer agent a compound of formula I, where x is 0 or 1, or a pharmaceutically acceptable salt thereof as defined above.

The invention also provides a method of treating ulcers or hypersecretion in a mammal in need of such treatment, which method comprises administering to said mammal an effective amount of a compound of formula I or a pharmaceutically acceptable salt thereof. The amount of compound I will depend on the activity of the compound and the needs of the mammal being treated. Doses may range from 0.1 to 30 mg/kg.

The following examples illustrate the invention:

## Example 1

5,6-Dihydro-2-(2-pyridylmethylthio)-4H-imidazo[4,5,1-ij]-quinoline

A solution of 5,6-dihydro-2-mercapto-4H-imidazo[4,5,1-ij]quinoline (1.9 g) in ethanol (30 ml) was heated to reflux and treated with 2-picolyl chloride, hydrochloride (2.5 g) and the solution was heated at reflux for 6 hours. A solid which formed on cooling was removed by filtration and dried to give the title compound as the dihydrochloride (2.8 g) mp 215°C decomp. (Found: C, 54.5; H, 5.0; N, 12.1. $C_{16}H_{16}N_3S{\cdot}2HCl$ requires C, 54.1; H, 5.1; N, 11.8%).

## Example 2

2-(2-Pyridylmethylsulphinyl)-5,6-dihydro-4H-imidazo[4,5,1-ij]quinoline

A solution of 5,6-dihydro-2-(2-pyridylmethylthio)-4H-imidazo[4,5,1-ij]quinoline hydrochloride (4.0 g) in water (10 ml) was basified with sodium carbonate solution and extracted into chloroform. The extracts were dried $(MgSO)_4$ and evaporated and the residue was dissolved in methylene chloride (50 ml) and cooled to −50°C. 3-chloroperoxybenzoic acid (2 g) was added with stirring and the solution was allowed to warm to −20°C when a further (0.5 g) of 3-chloroperoxybenzoic acid was added and the solution was kept at 6°C for 16 hours. It was then washed ($Na_2CO_3$ solution and brine), dried ($MgSO_4$) and evaporated to give an oil which was purified by chromatography on alumina with ethyl acetate as eluent and crystallised from di-isopropylether to give the title compound as a quarterhydrate (2.1 g) mp 100—102°C. (Found: C, 63.9; H, 5.1; N, 14.0. $C_{16}H_{15}N_3OS$, $\frac{1}{4}H_2O$ requires C, 63.7; H, 5.2; N, 13.9%);.

## Example 3

5,6-Dihydro-2-(2-(3-methylpyridyl)methylthio)-4-[H]-imidazo[4,5,1-ij]quinoline

A stirred suspension of 5,6-dihydro-2-mercapto-[4H]-imidazo[4,5,1-ij]quinoline (3.8 g) in ethanol (40 ml) at reflux was treated with 3-methylpicolylchloride, hydrochloride (3.6 g) and the mixture was heated for 4 hours. The solvent was removed by evaporation and the residue dissolved in water, basified with 2N NaOH and extracted with EtOAc. The extracts were dried ($MgSO_4$) and evaporated to give a solid which was recrystallised from cyclohexane to give the title compound (4.5 g, 75%) mp 106—8°C. (Found: C, 69.3; H, 5.9; N, 13.9; $C_{17}H_{17}N_3S$ requires C, 69.1; H, 5.8; N, 14.2%).

Example 4

2-(2-Pyridylmethylthio)-4[H]-imidazo[4,5,1-ij]quinoline

2-Picolyl chloride, hydrochloride (0.8 g, .005 mole) was added to a stirred solution of 2-mercapto-[4H]-imidazo[4,5,1-ij]quinoline (0.83 g, .004 mole) in ethanol (17 ml) at reflux. The mixture was heated at reflux for 4 hours and evaporated. The residue was dissolved in water, basified (2N NaOH) and extracted with ethyl acetate. The extracts were washed N/10 NaOH), dried ($MgSO_4$) and evaporated and the residue was recrystallised from cyclohexane to give the title compound (0.8 g, 65%) mp 109—10°C (Found: C, 68.95; H, 4.7; N, 14.8; $C_{16}H_{13}N_3S$ requires C, 68.8; H, 4.7; N, 15.05%).

Example 5

5,6-Dihydro-2-(2-(3-methylpyridyl)methylsulphinyl)[4H]-imidazo[4,5,1-ij]quinoline

To a solution of 5,6-Dihydro-2-(2-(3-methylpyridyl)methylthio)-4-[H]-imidazo[4,5,1-ij]quinoline (1 g, 0.0034 mole) in $CH_2Cl_2$ (50 ml) was added m-chloroperoxybenzoic acid (80%) (0.72 g, 0.0034 mole) at 0°C with stirring. The solution was stirred for 1 hour when t.l.c. ($SiO_2$/$Et_2O$ or EtOAc) showed starting material present and further m-chloroperoxybenzoic acid (0.1 g) was added. The solution was stirred at 0°C for ½ hour and allowed to warm to ambient temperature, washed with saturated $Na_2CO_3$ solution, $H_2O$ and brine, dried ($MgSO_4$) and evaporated. The residue was purified by chromatography on silica with ethyl acetate as eluent followed by trituration with ethyl acetate to give the title compound (0.6 g, 57%) mp 172—3°C. (Found: C 65.7; H, 5.6; N, 13.2. $C_{17}H_{17}N_3OS$ requires C, 65.6; H, 5.5; N, 13.5%).

Example 6

5,6-Dihydro-2-(2-(6-phenylpyridyl)methylthio)-[4H]-imidazo[4,5,1-ij]quinoline

5,6-Dihydro-2-mercapto-[4H]imidazo[4,5,1-ij]quinoline (1.8 g) was suspended in ethanol (25 ml) and a solution of NaOH (0.8 g) in water (5 ml) was added. The resulting solution was treated with 6-phenylpicolyl chloride, hydrochloride (2.4 g) and the mixture was heated at reflux for 1 hour. The reaction mixture was filtered and evaporated to give an oil which was dissolved in ethyl acetate. The solution was quickly dried ($MgSO_4$) and filtered and crystals allowed to form. These were removed by filtration and dried to give the title compound (2.2 g, 62%) mp 121—3°C. (Found: C, 74.0; H, 5.5; N, 11.8; $C_{22}H_{19}N_3S$ requires C, 73.9; H, 5.4; N, 11.8%).

Example 7

5,6-Dihydro-2-(2-(3,5-dimethylpyridyl)methylthio-[4H]-imidazo[4,5,1-ij]quinoline

A solution of 5,6-dihydro-2-mercapto-[4H]-imidazo-[4,5,1-ij]quinoline (1.9 g) in ethanol (30 ml), water (5 ml) and NaOH (0.4 g) was treated with 3,5-dimethylpicolyl chloride hydrochloride (1.9 g) at ambient temperature for 24 hours then at 60°C for 24 hours. The solution was filtered and allowed to stand when crystals formed which were removed by filtration and dried to give the title compound as the hydrochloride (1.2 g, 35%) mp 195—9°C. (Found: C, 62.0; H, 5.9; N, 11.9; $C_{18}H_{19}N_3S{\cdot}HCl$ requires C, 62.5; H, 5.8; N, 12.15%).

Example 8

5,6-Dihydro-2-(2-(3,5-dimethylpyridyl)methylsulphinyl-4H-imidazo[4,5,1-ij]quinoline

A solution of 5,6-dihydro-2-(2-(3,5-dimethylpyridyl)methylthio)-[4H]-imidazo[4,5,1-ij]quinoline (free base) (1.25 g) in $CH_2Cl_2$ (20 ml) was treated with m-chloroperoxybenzoic acid (0.9 g) at 0°C for 1 hour. The solution was washed ($Na_2CO_3$) dried ($MgSO_4$) and the products separated by chromatography on silica with ethyl acetate as eluent followed by recrystallisation from EtOAc-MeCH—$CHCl_3$ to give the title compound (2.6 g, 20%) mp 164—5°C. (Found: C, 66.2; H, 6.0; N, 12.9; $C_{18}H_{19}N_3OS$ requires C, 66.4; H, 5.9; N, 12.9%).

Example 9

5,6-Dihydro-2-[(2-phenylthiazol-4-yl)methylthio]-4H-imidazo[4,5,1-ij]quinoline

A solution of 5,6-dihydro-2-mercapto[4H]imidazo[4,5,1-ij]quinoline (2.04 g, 10.7 mmol) in 1 *N*-sodiumhydroxide (10.7 ml) and ethanol (10 ml) was treated with 4-chloromethyl-2-phenylthiazole (2.25 g, 10.7 mmol) and the resulting solution heated under reflux for 5 mins. The mixture was concentrated *in vacuo* and the aqueous mixture extracted with ethyl acetate (2 × 50 ml). The extracts were dried ($MgSO_4$) and evaporated *in vacuo* to give a yellow oil which crystallised on trituration with diethyl ether (50 ml) to give the title compound (3.62 g, 93%), mp 116—118°. (Found: C, 66.4; H, 5.0; N, 11.3; $C_{20}H_{17}N_3S_2$ requires C, 66.1; H, 4.7; N, 11.6%).

Example 10

A) 5,6-Dihydro-8-methoxy-4H-imidazo[4,5,1-ij]quinoline-2[1H]-thione

A suspension of 6-methoxy-8-nitroquinoline (20 g, 98 mmol) in methanol (200 ml) was hydrogenated over $PtO_2$ (0.5 g) at 50 p.s.i. for 2.5 hours. The solution was treated with acetic acid (11.5 ml, 200 mmol) and $PtO_2$ (0.5 g) and hydrogenated at 50 p.s.i. for 18 hours. The catalyst was removed by filtration and the solution evaporated *in vacuo* to give an oil. The residue was basified with 1 *N*-sodium hydroxide (200 ml) and the aqueous mixture extracted with diethyl ether (2 × 200 ml). The extracts were dried ($MgSO_4$) and evaporated *in vacuo* to give crude 8-amino-1,2,3,4-tetrahydro-6-methoxyquinoline as a red oil.

The oil was dissolved in methanol (100 ml) and the solution treated with $CS_2$ (10 ml, 167 mmol), heated under reflux for 18 hours, cooled to room temperature, and evaporated *in vacuo* to give a brown solid. The solid was dissolved in warm 1 *N*-sodium hydroxide (100 ml) and the cold solution washed with di-isopropyl ether (2 × 200 ml). The ethereal washings were extracted with 0.1 *N*-sodium hydroxide (100 ml). The aqueous solution and extract were combined, heated on a steam-bath, decolourised with activated charcoal, filtered, cooled to 0°C., and neutralised with stirring with acetic acid (10 ml). The precipitate was filtered, washed with copious amounts of water and dried *in vacuo* to give 5,6-dihydro-8-methoxy-4H-imidazo[4,5,1-ij]quinoline-2[1H]-thione (18.2 g, 84%) as light brown crystals, mp 188—192°C. (Found: C, 59.8; H, 5.5; N, 12.4, $C_{11}H_{12}N_2OS$ requires C, 60.0; H, 5.5; N, 12.7%).

B) 5,6-Dihydro-8-methoxy-2-[(6-phenylpyridin-2-yl)-methylthio]-4H-imidazo[4,5,1-ij]quinoline

2-Chloromethyl-6-phenylpyridine hydrochloride (1.942 g, 8.1 mmol) was added to a solution of 5,6-dihydro-8-methoxy-4H-imidazo[4,5,1-ij]quinoline-2[1H]-thione (1.78 g, 8.1 mmol) in ethanol (16 ml) and 1 *N*-sodium hydroxide (16.2 ml). The mixture was heated under reflux for 15 seconds and cooled to room temperature. The ethanol was removed by evaporation *in vacuo* and the aqueous mixture extracted with dichloromethane (2 × 150 ml). The extracts were dried ($MgSO_4$) and evaporated *in vacuo* to give a brown solid which was recrystallised from MeOH to give the title compound as the quarter hydrate (2.77 g, 88%), mp 134—135°C. (Found: C, 70.5; H, 5.8; N 10.4; $C_{23}H_{21}N_3OS\frac{1}{4}H_2O$ requires C, 70.5; H, 5.5; N, 10.7%).

## Example 11

5,6-Dihydro-2-(quinolin-2-ylmethylthio)-4H-imidazo[4,5,1-ij]quinoline

A solution of 5,6-dihydro-4*H*-imidazo[4,5,1-ij]quinoline-2-(1*H*)-thione (2.02 g, 11.6 mmol) in ethanol (25 ml) and 1 *N*-sodium hydroxide was treated with 2-(chloromethyl)quinoline hydrochloride (2.48 g, 11.6 mmol) heated to reflux, cooled to room temperature, and the ethanol removed by evaporation *in vacuo.* The aqueous mixture was filtered and the precipitate washed with water (5 × 50 ml), dried *in vacuo,* and recrystallised from cyclohexane — EtOAc to give 5,6-dihydro-2-(quinolin-2-ylmethylthio)-4H-imidazo[4,5,1-ij]quinoline (2.13 g; 55%) as yellow crystals mp 125—126°C. (Found: C, 72.8; H, 5.3; N, 12.4; $C_{20}H_{17}N_3S$ requires C, 72.5; H, 5.2; N, 12.7%).

## Example 12

5,6-Dihydro-2-(3-pyridylmethylthio)-4H-imidazo[4,5,1-ij]quinoline

Following the procedure of Example 1, 5,6-dihydro-2-mercapto-4H-imidazo[4,5,1-ij]quinoline (1.9 g) in ethanol (30 ml) is treated with 3-picolyl chloride, hydrochloride (2.5 g) to obtain the title compound as the dihydrochloride.

## Example 13

5,6-Dihydro-2-(4-pyridylmethylthio-4H-imidazo[4,5,1-ij]quinoline

Following the procedure of Example 1, 5,6-dihydro-2-mercapto-4H-imidazo[4,5,1-ij]quinoline (1.9 g) in ethanol (30 ml) is treated with 4-picolyl chloride, hydrochloride (2.5 g) to obtain the title compound as the hydrochloride. Free base has mp 105—107°C.

## Example 14

Following the procedure of Example 1 the following compounds are prepared.

Starting Materials                                        Final Product

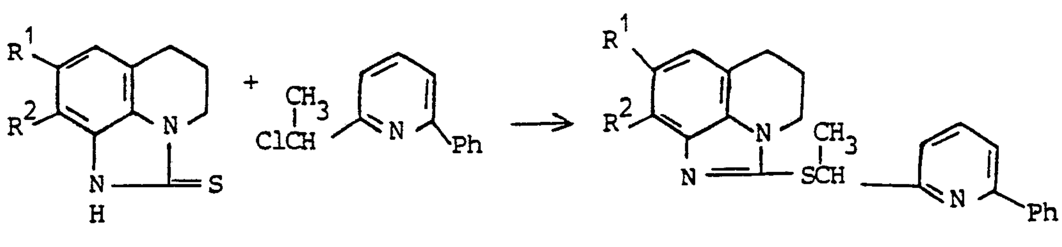

a) $R^1$ and $R^2$ = H

b) $R^1$ = H; $R^2$ = Cl

c) $R^1$ = CN; $R^2$ = H

d) $R^1$ = $NO_2$; $R^2$ = H

e) $R^1$ = Cl; $R^2$ = H

Example 15

Following the procedure of Example 1 the following compounds are prepared.

Starting Materials | Final Product

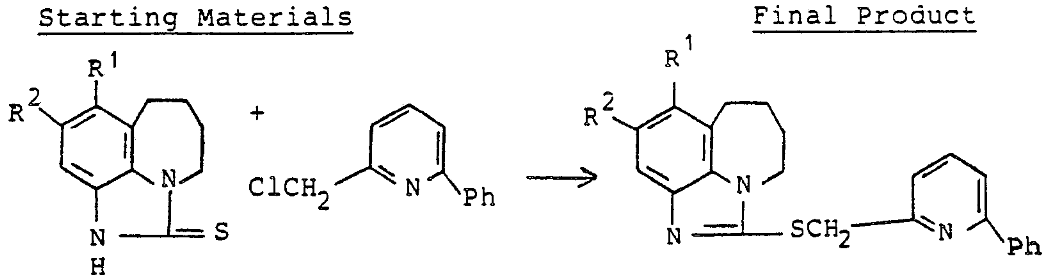

a) $R^1$ and $R^2$ = H

b) $R^1$ = Cl; $R^2$ = H

c) $R^1$ = CN; $R^2$ = H

d) $R^1$ = H; $R^2$ = Cl

e) $R^1$ = H; $R^2$ = $NO_2$

Example 16

Following the procedure of Example 2 the following sulphinyl compounds are prepared.

Starting Materials | Final Product

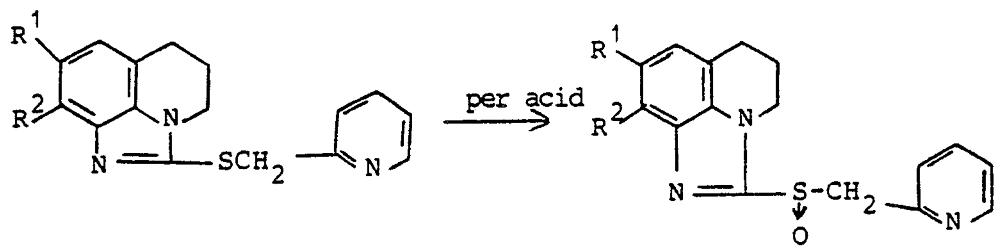


a) $R^1$ = MeO; $R^2$ = H

b) $R^1$ = CN; $R^2$ = H

c) $R^1$ = H; $R^2$ = MeO

d) $R^1$ = CN; $R^2$ = MeO

Example 17

Following the procedure of Example 9 the following compounds are prepared.

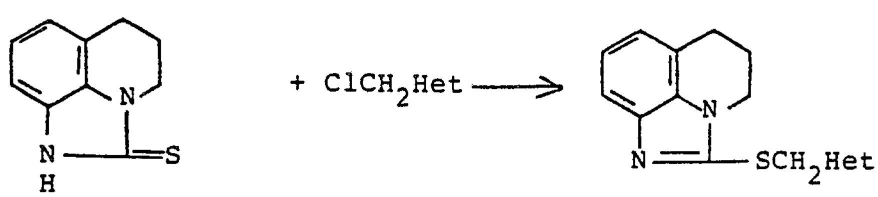

a) Het = benzimidazol-2-yl

b) Het = benzothiazol-2-yl

c) Het = pyrimid-2-yl

Example 18

Following the procedure of Example 2 the following sulphinyl compounds are prepared.

Starting Material | Final Product

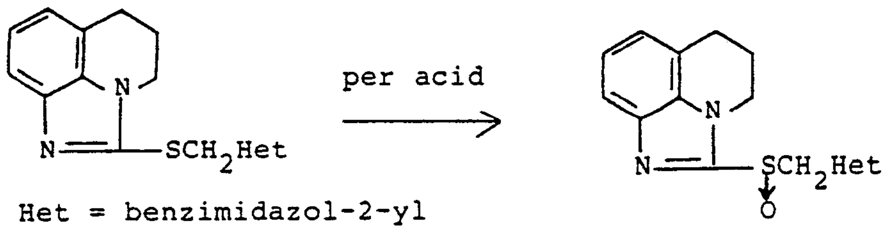


a) Het = benzimidazol-2-yl

b) Het = pyrimid-2-yl

Example 19

Following the general procedures of Examples 11 and 13 respectively the following compounds are prepared.

a) 5,6-Dihydro-8-methoxy-2-(quinolin-2-ylmethylthio)-4H-imidazo-[4,5,1-ij]quinoline mp 89—91°C.

b) 5,6-Dihydro-8-methoxy-2-(4-pyridylmethylthio-4H-imidazo[4,5,1-ij]-quinoline mp 99—101°C.

Pharmacological Test Results

| Compound | Stress Induced Erosion Senay & Levine | | Anti-Secretory (Shay *et al*) | | % Inhibition to Stimulation by | |
|---|---|---|---|---|---|---|
| | Dose mg/kg | Inhibition | Dose mg/kg | % Change in volume | DBcAMP at $10^{-4}$ | $K^+$ at $10^{-4}$ |
| Example 1 | 100 | 69 | 30 | N/A | 34 | -215 |
| Example 2 | 100 | 60 | 30 | N/A | 97 | 65 |
| Example 3 | 100 | 68 | 30 | N/A | N/A | -109 |
| Example 4 | | N/T | 30 | N/A | 16.7 | 35.5 |
| Example 5 | | N/T | 30 | N/A | 66 | N/A |
| Example 6 | 100 | 58% | 30 | N/A | 36 | 182 |
| Example 7 | 100 | N/A | 30 | N/A | 70 | 74 |
| Example 8 | | N/T | 30 | N/T | 48 | N/A |
| Example 9 | 100 | N/A | 30 | N/A | 75 | 122 |
| Example 10B | 100 | N/A | 30 | N/A | 98 | 136 |
| Example 11 | 100 | N/A | 30 | 22 | 62 | 147 |

N/A = No significant activity

N/T = Not tested

**Claims for the Contracting States: BE CH DE FR IT LI LU NL SE**

1. A compound of formula I

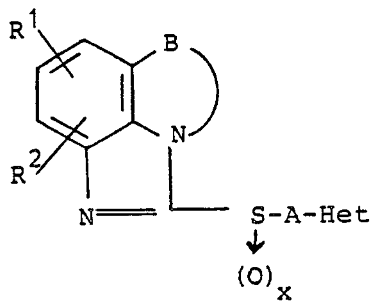

(I)

wherein

A is a $C_1$—$C_4$ straight or branched alkylene chain which may be saturated or unsaturated,

B is a $C_2$—$C_4$ straight or branched alkylene chain which may be saturated or unsaturated,

$R^1$ and $R^2$ are the same or different and are hydrogen, $C_1$—$C_6$-alkyl, $C_1$—$C_6$-alkoxy, $C_1$—$C_6$-alkoxy($C_1$—$C_6$)alkyl, $C_1$—$C_6$-hydroxyalkyl, hydroxy, halogen, nitro, carboxy, carboxylic ester, carbamoyl, carbamoyloxy, cyano, $C_2$—$C_7$ alkanoyl, $C_2$—$C_7$ alkanoylamino or trifluoromethyl,

Het is a heterocyclic group chosen from imidazolyl, imidazolinyl, benzimidazolyl, thiazolyl, thiazolinyl, quinolyl, piperidyl, pyridyl, benzothiazolyl and pyrimidyl, any of which heterocyclic groups may be substituted by one or more of the following; halogen, $C_1$—$C_6$-alkoxy, $C_7$—$C_{12}$-aralkoxy, hydroxy, $C_1$—$C_6$ hydroxyalkyl, $C_1$—$C_6$ alkoxy($C_1$—$C_6$)alkyl, trifluoromethyl, $C_1$—$C_6$ alkyl, phenyl or $C_7$—$C_{12}$ aralkyl, or disubstituted by loweralkylenedioxy of 1 to 6 carbon atoms and the phenyl or aryl portion an aralkyl group may be substituted by any of the foregoing substituents and x is 0 or 1, and pharmaceutically acceptable salts thereof.

2. A compound as claimed in Claim 1, wherein Het is pyridyl, quinolyl or thiazoly which may be substituted by one or more of the following; halogen, $C_1$—$C_6$-alkoxy, $C_7$—$C_{12}$-aralkoxy, hydroxy, $C_1$—$C_6$ hydroxyalkyl $C_1$—$C_6$ alkoxy-$C_1$—$C_6$ alkyl, trifluoromethyl, $C_1$—$C_6$ alkyl, phenyl or $C_7$—$C_{12}$ aralkyl, or disubstituted by loweralkylenedioxy of 1 to 6 carbon atoms and the phenyl or aryl portion of the aralkyl group may be substituted by any of the foregoing substituents.

3. A compound as claimed in Claim 1 or Claim 2, wherein Het is joined as the 2-position.

4. A compound of formula I as claimed in Claim 1 and having formula IA

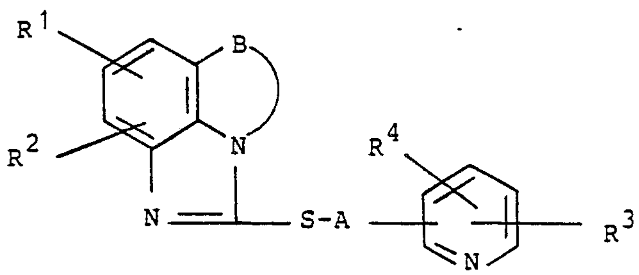

(Ia)

wherein A, $R^1$ and $R^2$ are as defined in Claim 1 in connection with formula I, $R^3$ and $R^4$ are the same or different and are hydrogen, $C_1$—$C_6$ alkyl or phenyl which may be substituted by any of the substituents described for Het Claim 1 and one of $R^3$ and $R^4$ is other than hydrogen, and pharmaceutically acceptable salts thereof.

5. A compound as claimed in any one of claims 1—4, wherein A is $CH_2$.

6. A compound as claimed in any one of the preceding claims wherein $R^1$ and $R^2$ are selected from hydrogen, $C_1$—$C_6$-alkyl and $C_1$—$C_6$ alkoxy.

7. A compound as claimed in any one of claims 1—5 wherein Het is 2-(6-phenyl-pyridyl).

8. A compound selected from

5,6-dihydro-2-(2-pyridylmethylthio)-4H-imidazo[4,5,1-ij]quinoline or a pharmaceutically acceptable salt thereof.

2-(2-pyridylmethylsulphinyl)-5,6-dihydro-4H-imidazo[4,5,1-ij]quinoline or a pharmaceutically acceptable salt thereof.

5,6-dihydro-2-(2-(3-methylpyridyl)methylthio)-4H-imidazo[4,5,1-ij]quinoline or a pharmaceutically acceptable salt thereof.

2-(2-pyridylmethylthio)-4H-imidazo[4,5,1-ij]quinoline or a pharmaceutically acceptable salt thereof.

5,6-dihydro-2-(2-(3-methylpyridyl)methylsulphinyl)-4H-imidazo[4,5,1-ij]quinoline or a pharmaceutically acceptable salt thereof.

5,6-dihydro-2-(2-(6-phenylpyridyl)methylthio)-4H-imidazo[4,5,1-ij]quinoline or a pharmaceutically acceptable salt thereof.

5,6-dihydro-2-(2-3,5-dimethylpyridyl)methylthio)-4H-imidazo[4,5,1-ij]quinoline or a pharmaceutically acceptable salt thereof.

5,6-dihydro-2-(2-(3,5-dimethylpyridyl)methylsulphinyl)-4H-imidazo[4,5,1-ij]quinoline or a pharmaceutically acceptable salt thereof.

5,6-dihydro-2-(6-phenyl-2-pyridylmethylthio)-4H-imidazo[4,5,1-ij]quinoline or a pharmaceutically acceptable salt thereof.

5,6-dihydro-2-(3-pyridylmethylthio)-4H-imidazo[4,5,1-ij]quinoline or a pharmaceutically acceptable salt thereof.

5,6-dihydro-2-(4-pyridylmethylthio)-4H-imidazo[4,5,1-ij]quinoline or a pharmaceutically acceptable salt thereof.

9. 5,6-Dihydro-2-(2-quinolylmethylthio)-4H-imidazo[4,5,1-ij]quinoline or a pharmaceutically acceptable salt thereof.

10. 5,6-Dihydro-2-[(2-phenylthiozol-4-yl)methylthio]-4H-imidazo[4,5,1-ij]quinoline or a pharmaceutically acceptable salt thereof.

11. A compound as claimed in any one of the preceding claims for use as a pharmaceutical.

12. A compound as claimed in any one of claims 1—10, for use as an anti-ulcer agent or in treating hyper-secretion.

13. A pharmaceutical composition comprising a compound as claimed in any one of claims 1—10, and a pharmaceutically acceptable carrier.

14. A pharmaceutical composition as claimed in Claim 13 in unit dosage form.

**Claims for the Contracting State: AT**

1. A process for preparing a compound of formula I

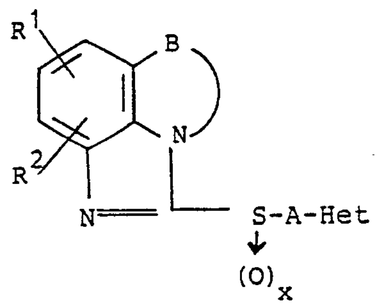

(I)

wherein

A is a $C_1$—$C_4$ straight or branched alkylene chain which may be saturated or unsaturated,

B is a $C_2$—$C_4$ straight or branched alkylene chain which may be saturated or unsaturated,

$R^1$ and $R^2$ are the same or different and are hydrogen $C_1$—$C_6$ alkyl, $C_1$—$C_6$ alkoxy, $C_1$—$C_6$ alkoxy[$C_1$—$C_6$]alkyl, $C_1$—$C_6$-hydroxyalkyl, hydroxy, halogen, nitro, carboxy, carboxylic ester, carbamoyl, carbamoyloxy, cyano, $C_2$—$C_7$-alkanoyl, $C_2$—$C_7$-alkanoylamino or trifluoromethyl,

Het is a heterocyclic group chosen from imidazolyl, imidazolinyl, benzimidazolyl, thiazolyl, thiazolinyl, quinolyl, piperidyl, pyridyl, benzothiazolyl and pyrimidyl, any of which heterocyclic groups may be substituted by one or more of the following; halogen, $C_1$—$C_6$ alkoxy, $C_7$—$C_{12}$ aralkoxy, hydroxy, $C_1$—$C_6$ hydroxyalkyl, $C_1$—$C_6$ alkoxy($C_1$—$C_6$)alkyl, trifluoromethyl, $C_1$—$C_6$ alkyl, phenyl, $C_7$—$C_{12}$ aralkyl, or disubstituted by loweralkylenedioxy of 1 to 6 carbon atoms, and the phenyl or aryl portion of an aralkyl group may be substituted by any of the foregoing substituents and x is 0 or 1, and pharmaceutically acceptable salts thereof, characterised in that:

A) a compound of Formula II

Het-A-Hal (II)

wherein Het and A are as defined above and Hal is a halogen atom is reacted with a thiol of Formula III, or an alkali metal salt thereof

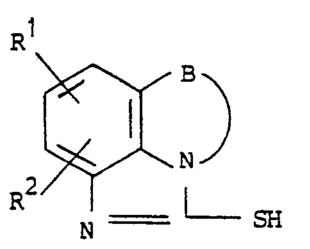

(III)

wherein B, $R^1$ and $R^2$ are as defined above; or

B) a compound of Formula IIa

Het-A-SH (IIa)

or an alkali metal salt thereof, wherein Het and A are as defined above is reacted with a halide of Formula IIIa

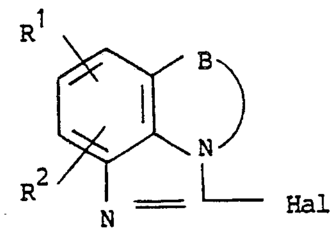

(IIIa)

wherein B, $R^1$, $R^2$ and Hal are as defined above; and

optionally where a compound of Formula I in which x is 1 is desired oxidising the product to obtain a compound of Formula I in which x is 1; or

C) a compound of Formula IV

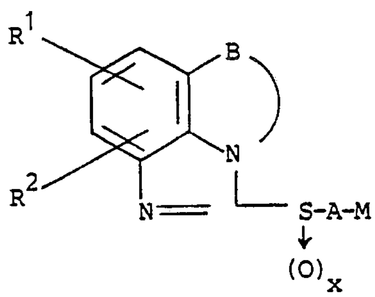

(IV)

wherein $R^1$, $R^2$, A, B and x are as defined above and M is sodium, potassium or lithium, is reacted with a compound of Formula

Het-Q (V)

where Het is as defined above and Q is a leaving group such as a reactive esterified hydroxy group or a halogen and if desired converting a product where x is 0 or 1 to a pharmaceutically acceptable salt.

2. A process for preparing a compound of Formula I as defined in Claim 1, or a pharmaceutically acceptable salt thereof, wherein x is 1, characterised in that a corresponding compound of formula I wherein x is 0, or a pharmaceutically acceptable salt thereof, is oxidised and if desired the product is isolated as a free base or a pharmaceutically acceptable salt.

3. A process as claimed in Claim 1, characterised in that a starting material is used in which Het is pyridyl, quinolyl or thiazolyl which may be substituted by one or more of the following; halogen, $C_1$—$C_6$ alkoxy, $C_7$—$C_{12}$ aralkoxy, hydroxy, hydroxy($C_1$—$C_6$)alkyl, $C_1$—$C_6$ alkoxy($C_1$—$C_6$)alkyl, trifluoromethyl, $C_1$—$C_6$ alkyl, phenyl $C_7$—$C_{12}$ aralkyl, or disubstituted by loweralkylenedioxy of 1 to 6 carbon atoms and the phenyl or aryl portion of the aralkyl group may be substituted by any of the foregoing substituents.

4. A process as claimed in Claim 1, Claim 2 or Claim 3, characterised in that a starting material is used in which A is $CH_2$.

5. A process as claimed in Claim 3, characterised in that a starting material is used in which Het is

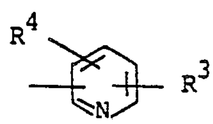

wherein $R^3$ and $R^4$ are the same or different and are hydrogen, $C_1$—$C_6$ alkyl or phenyl which may be substituted by any of the substituents described for Het in Claim 1, and one of $R^3$ and $R^4$ is other than hydrogen, and pharmaceutically acceptable salts thereof.

6. A process as claimed in Claim 5, characterised in that a starting material is used in which Het is 2-(6-phenyl-pyridyl).

7. A process as claimed in Claim 1, characterised in that a starting material is used in which Het is 2-phenylthiazol-4-yl, or 2-quinolyl.

8. A process for preparing a pharmaceutical composition characterised in that a compound of formula I as defined in Claim 1 is prepared by a process as claimed in any one of claims 1—7 and is then mixed with a pharmaceutical carrier and the resulting composition is brought into a form suitable for therapeutic administration.

9. A process as claimed in Claim 8, characterised in that the composition is formulated as tablets, capsules or other unit dosage form.

**Patentansprüche für die Vertragsstaaten: BE CH DE FR IT LI LU NL SE**

1. Eine Verbindung der Formel (I)

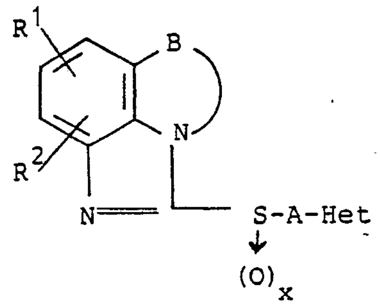

(I)

worin

A eine gerade oder verzweigte $C_1$—$C_4$-Alkylenkette ist, die gesättigt oder ungesättigt sein kann;

B eine gerade oder verzweigte $C_2$—$C_4$-Alkylenkette ist, die gesättigt oder ungesättigt sein kann; $R^1$ und $R^2$ gleich oder verschieden sind und Wasserstoff, $C_1$—$C_6$-Alkyl, $C_1$—$C_6$-Alkoxy, ($C_1$—$C_6$)-Alkoxy($C_1$—$C_6$)-alkyl, $C_1$—$C_6$-Hydroxyalkyl, Hydroxy, Halogen, Nitro, Carboxy, Carbonsäureester, Carbamoyl, Carbamoyloxy, Cyano, $C_2$—$C_7$-Alkanoylamino oder Trifluormethyl bedeuten;

Het eine heterocyclische Gruppe ausgewählt aus Imidazolyl, Imidazolinyl, Benzimidazolyl, Thiazolyl, Thiazolinyl, Chinolyl, Piperidyl, Pyridyl, Benzothiazolyl und Pyrimidyl darstellt, wobei jede heterocyclische Gruppe durch eines oder mehrere der folgenden substituiert sein kann; Halogen, $C_1$—$C_6$-Alkoxy, $C_7$—$C_{12}$-Aralkoxy, Hydroxy, $C_1$—$C_6$-Hydroxyalkyl, $C_1$—$C_6$-Alkoxy-($C_1$—$C_6$)alkyl, Trifluormethyl, $C_1$—$C_6$-Alkyl, Phenyl oder $C_7$—$C_{12}$-Aralkyl oder durch nied. Alkylendioxy mit 1 bis 6 C-Atomen disubstituiert sein kann, und der Phenyl- oder Arylteil einer Aralkylgruppe durch einen der obigen Substituenten substituiert sein kann, und x Null oder 1 ist, und pharmazeutisch annehmbare Salze hievon.

2. Verbindung, wie in Anspruch 1 beansprucht, worin Het Pyridyl, Chinolyl oder Thiazolyl ist, die durch einen oder mehrere der folgenden substituiert sein können: Halogen, $C_1$—$C_6$-Alkoxy, $C_7$—$C_{12}$-Aralkoxy, Hydroxy, $C_1$—$C_6$-Hydroxyalkyl, $C_1$—$C_6$-Alkoxy-$C_1$—$C_6$-alkyl, Trifluormethyl, $C_1$—$C_6$-Alkyl, Phenyl oder $C_7$—$C_{12}$-Aralkyl oder durch nied. Alkylendioxy mit 1 bis 6 C-Atomen disubstituiert sein können und der Phenyl- oder Arylteil der Aralkylgruppe durch einen der obigen Substituenten substituiert sein kann.

3. Verbindung, wie in Anspruch 1 oder 2 beansprucht, worin Het in Stellung 2 gebunden ist.

4. Verbindung der Formel (I), wie in Anspruch 1 beansprucht, die die Formel (IA)

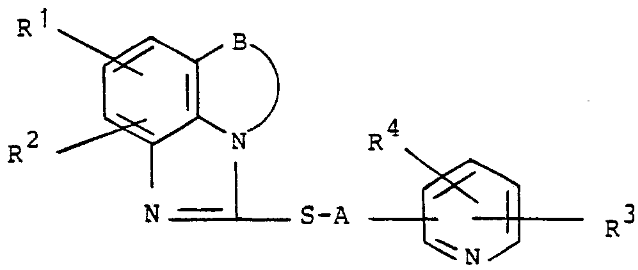

(Ia)

aufweist, worin A, $R^1$ und $R^2$ wie in Anspruch 1 in Verbindung mit Formel (I) definiert sind, $R^3$ und $R^4$ gleich oder verschieden sind und Wasserstoff, $C_1$—$C_6$-Alkyl oder Phenyl bedeuten, die durch einen der für Het in Anspruch 1 beschriebenen Substituenten substituiert sein können, und eines von $R^3$ und $R^4$ eine andere Bedeutung als Wasserstoff hat, und pharmazeutisch annehmbare Salze hievon.

5. Verbindung, wie in einem der Ansprüche 1 bis 4 beansprucht, worin a $CH_2$ ist.

6. Verbindung, wie in einem der vorhergehenden Ansprüche beansprucht, worin $R^1$ und $R^2$ ausgewählt sind aus Wasserstoff, $C_1$—$C_6$-Alkyl und $C_1$—$C_6$-Alkoxy.

7. Verbindung, wie in einem der Ansprüche 1 bis 5 beansprucht, worin Het 2-(6-Phenylpyridyl) ist.

8. Verbindung ausgewählt aus

5,6-Dihydro-2-(2-pyridylmethylthio)-4H-imidazo[4,5,1-ij]chinolin oder einem pharmazeutisch annehmbaren Salz hievon,

2-(2-Pyridylmethylsulfinyl)-5,6-dihydro-4H-imidazo[4,5,1-ij]chinolin oder einem pharmazeutisch annehmbaren Salz hievon,

5,6-Dihydro-2-[2-(3-methylpyridyl)-methylthio]-4-[H]-imidazo[4,5,1-ij]chinolin oder einem pharmazeutisch annehmbaren Salz hievon,

2-(2-Pyridylmethylthio)-4[H]-imidazo[4,5,1-ij]chinolin oder einem pharmazeutisch annehmbaren Salz hievon,

5,6-Dihydro-2-[2-(3-methylpyridyl)-methylsulfinyl)-[4H]-imidazo[4,5,1-ij]chinolin oder einem pharmazeutisch annehmbaren Salz hievon,

5,6-Dihydro-2-[2-(6-phenylpyridyl)-methylthio]-[4H]-imidazo[4,5,1-ij]chinolin oder einem pharmazeutisch annehmbaren Salz hievon,

5,6-Dihydro-2-(2-(3,5-dimethylpyridyl)-methylthio)-[4H]-imidazo[4,5,1-ij]chinolin oder einem pharmazeutisch annehmbaren Salz hievon,

5,6-Dihydro-2-[2-(3,5-dimethylpyridyl)-methylsulfinyl]-[4H]-imidazo[4,5,1-ij]chinolin oder einem pharmazeutisch annehmbaren Salz hievon,

5,6-Dihydro-2-(6-phenyl-2-pyridylmethylthio)-4H-8-methoxyimidazo[4,5,1-ij]chinolin oder einem pharmazeutisch annehmbaren Salz hievon,

5,6-Dihydro-2-(3-pyridylmethylthio)-4H-imidazo[4,5,1-ij]chinolin oder einem pharmazeutisch annehmbaren Salz hievon,

5,6-Dihydro-2-(4-pyridylmethylthio-4H-imidazo[4,5,1-ij]chinolin oder einem pharmazeutisch annehmbaren Salz hievon,

9. 5,6-Dihydro-2-(2-chinolylmethylthio)-4H-imidazo[4,5,1-ij]chinolin oder ein pharmazeutisch annehmbares Salz hievon.

10. 5,6-Dihydro-2-[(2-phenylthiazol-4-yl)-methylthio]-4H-imidazo[4,5,1-ij]chinolin oder ein pharmazeutisch annehmbares Salz hievon.

11. Verbindung, wie in einem der vorhergehenden Ansprüche beansprucht, zur Verwendung als Pharmazeutikum.

12. Vebindung, wie in einem der Ansprüche 1 bis 10 beansprucht, zur Verwendung als Antigeschwürmittel oder beim Behandeln von Hypersekretion.

13. Pharmazeutische Zussammensetzung umfassend eine Verbindung, wie in einem der Ansprüche 1 bis 1 beansprucht, und einen pharmazeutisch annehmbaren Träger.

14. Pharmazeutische Zusammensetzung, wie in Anspruch 13 beansprucht, in einheitsdosierungsform.

**Patentansprüche für den Vertragsstaat: AT**

1. Verfahren zum Herstellen einer Verbindung Formel (I)

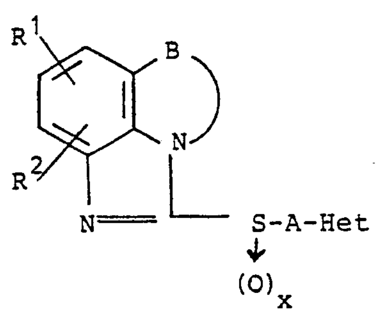

(I)

worin

A eine gerade oder verzweigte $C_1$—$C_4$-Alkylenkette ist, die gesättigt oder ungesättigt sein kann;

B eine gerade oder verzweigte $C_2$—$C_4$-Alkylenkette ist, die gesättigt oder ungesättigt sein kann; $R^1$ und $R^2$ gleich oder verschieden sind und Wasserstoff, $C_1$—$C_6$-Alkyl, $C_1$—$C_6$-Alkoxy, ($C_1$—$C_6$)-Alkoxy($C_1$—$C_6$)-alkyl, $C_1$—$C_6$-Hydroxyalkyl, Hydroxy, Halogen, Nitro, Carboxy, Carbonsäureester, Carbamoyl, Carbamoyloxy, Cyano, ($C_2$—$C_7$)-Alkanoyl, 4$C_2$—$C_7$-Alkanoylamino oder Trifluormethyl bedeuten;

Het eine heterocyclische Gruppe ausgewählt aus Imidazolyl, Imidazolinyl, Benzimidazolyl, Thiazolyl, Thiazolinyl, Chinolyl, Piperidyl, Pyridyl, Benzothiazolyl und Pyrimidyl darstellt, wobei jede heterocyclische Gruppe durch eines oder mehrere der folgenden substituiert sein kann; Halogen, $C_1$—$C_6$-Alkoxy, $C_7$—$C_{12}$-Aralkoxy, Hydroxy, $C_1$—$C_6$-Hydroxyalkyl, $C_1$—$C_6$-Alkoxy-($C_1$—$C_6$)alkyl, Trifluormethyl, $C_1$—$C_6$-Alkyl, Phenyl, $C_7$—$C_{12}$-Aralkyl oder durch nied. Alkylendioxy mit 1 bis 6 C-Atomen disubstituiert sein kann, und der Phenyl- oder Arylteil einer Aralkylgruppe durch einen der obigen Substituenten substituiert sein kann, und x Null oder 1 ist, und pharmazeutisch annehmbare Salzen hievon, dadurch gekennzeichnet, daß

A) eine Verbindung der Formel (II)

Het-A-Hal (II)

worin Het und A wie oben definiert sind und Hal ein Hal ein Halogenatom ist, mit einem Thiol der Formel (III) oder einem Alkalimetallsalz hievon.

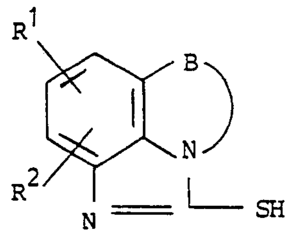

(III)

worin B, $R^1$ und $R^2$ wie oben definiert sind, umgesetzt wird; oder

B) eine Verbindung der Formel

Het-A-SH (IIa)

oder ein Alkalimetallsalz hievon, worin Het und A wie oben definiert sind, mit einem Halogenix der Formel (IIIa)

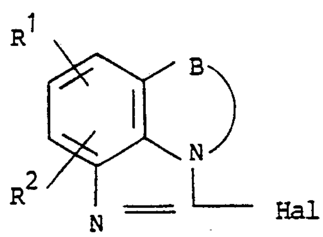

(IIIa)

worin B, $R^1$, $R^2$ und Hal wie oben definiert sind, umgesetzt wird; und

gegenbenenfalls, wenn eine Verbindung der Formel (I), worin x 1 ist, erwünscht ist, das Produkt zu einer Verbindung der Formel (I), worin x 1 ist, oxidiert wird; oder

C) eine Verbindung der Formel (IV)

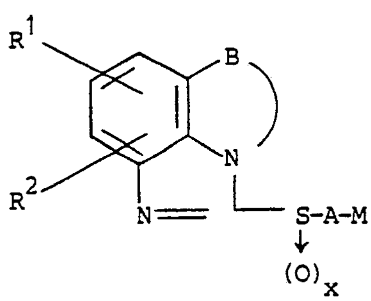

(IV)

worin $R^1$, $R^2$, A, B und x wie oben definiert sind und M Natrium, Kalium oder Lithium ist, mit einer Verbindung der Formel

Het-Q (V)

worin Het wie oben definiert ist und Q eine abspaltbare Gruppe, wie eine reaktive veresterte Hydroxygruppe oder Halogen, ist, umgesetzt wird und, wenn gewünscht, ein Produkt, worin x Null oder 1 ist, in ein pharmazeutisch annehmbares Salz übergeführt wird.

2. Verfahren zum Herstellen einer Verbindung der Formel (I), wie in Anspruch 1 definiert, oder eines pharmazeutisch annehmbaren Salzes hievon, worin x 1 ist, dadurch gekennzeichnet, daß eine entsprechende Verbindung der Formel (I), worin x Null ist, oder ein pharmazeutisch annehmbares Salz hievon oxidiert und, wenn gewünscht, das Produkt als freie Base oder pharmazeutisch annehmbares Salz isoliert wird.

3. Verfahren, wie in Anspruch 1 beansprucht, dadurch gekennzeichnet, daß ein Ausgangsmaterial verwendet wird, worin Het Pyridyl, Chinolyl oder Thiazolyl ist, die durch einen oder mehrere der folgenden substituiert sein können: Halogen, $C_1$—$C_6$-Alkoxy, $C_7$—$C_{12}$-Aralkoxy, Hydroxy, Hydroxy-($C_1$—$C_6$)alkyl, $C_1$—$C_6$-Alkoxy-$C_1$—$C_6$-alkyl, Trifluormethyl, $C_1$—$C_6$-Alkyl, Phenyl, $C_7$—$C_{12}$-Aralkyl oder durch nied. Alkylendioxy mit 1 bis 6 C-Atomen disubstituiert sein können, und der Phenyl- oder Arylteil der Aralkylgruppe durch einen der obigen Substituenten substituiert sein kann.

4. Verfahren, wie in Anspruch 1, 2 oder 3 beansprucht, dadurch gekennzeichnet, daß ein Ausgangsmaterial verwendet wird, worin A $CH_2$ ist.

5. Verfahren, wie in Anspruch 3 beansprucht, dadurch gekennzeichnet, daß ein Ausgangsmaterial verwendet wird, worin Het

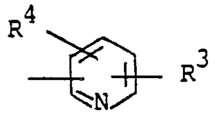

ist, wobei $R^3$ und $R^4$ gleich oder verschieden sind und Wasserstoff, $C_1$—$C_6$-Alkyl oder Phenyl bedeuten, das durch einen der für Het in Anspruch 1 beschriebenen Substituenten substituiert sein kann, und eines von $R^3$ und $R^4$ eine andere Bedeutung als Wasserstoff hat, und pharmazeutisch annehmbare Salze hievon.

6. Verfahren, wie in Anspruch 5 beansprucht, dadurch gekennzeichnet, daß ein Ausgangsmaterial verwendet wird, worin Het 2-(6-Phenylpyridyl) ist.

7. Verfahren, wie in Anspruch 1 beansprucht, dadurch gekennzeichnet, daß ein Ausgangsmaterial verwendet wird, worin Het 2-Phenylthiazol-4-yl oder 2-Chinolyl ist.

8. Verfahren zum Herstellen einer pharmazeutischen Zusammensetzung, dadurch gekennzeichnet, daß eine Verbindung der Formel (I), wie in Anspruch 1 definiert, nach einem Verfahren, wie in einem der Ansprüche 1 bis 7 beansprucht, hergestellt und dann mit einem pharmazeutischen Träger gemischt wird und die erhaltene Zusammensetzung in eine für therapeutische Verabreichung geeignete Form gebracht wird.

9. Verfahren, wie in Anspruch 8 beansprucht, dadurch gekennzeichnet, daß die Zusammensetzung als Tabletten, Kapseln oder andere Einheitsdosierunsform formuliert wird.

**Revendications pour les Etats Contactants: BE CH DE FR IT LI LU NL SE**

1. Composé de formule I

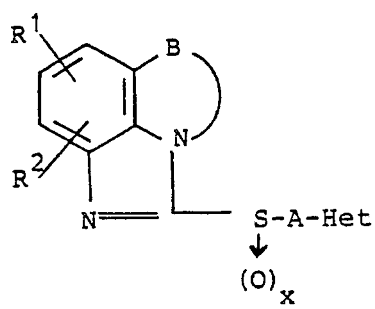

(I)

où

A est une chaîne alcoylène droite ou ramifiée en $C_1$—$C_4$ qui peut être saturée ou insaturée,

B est une chaîne alcoylène droite ou ramifiée en $C_2$—$C_4$ qui peut être saturée ou insaturée,

$R^1$ et $R^2$ sont identiques ou différents et sont un atome d'hydrogène ou radical $C_1$—$C_6$-alcoyle, $C_1$—$C_6$-alcoxy, $C_1$—$C_6$-alcoxy($C_1$—$C_6$)alcoyle, $C_1$—$C_6$-hydroxyalcoyle, hydroxyle, halogéno, nitro, carboxyle, ester carboxylique, carbamoyle, carbamoyloxy, cyano, $C_2$—$C_7$-alcanoyle, $C_2$—$C_7$-alcanoylamino ou trifluorométhyle,

Het est un radical hétérocyclique choise parmi imidazolyle, imidazolinyle, benzimidazolyle, thiazolyle, thiazolinyle, quinoléyle, pipéridyle, pyridyle, benzothiazolyle et pyrimidyle, l'un quelconque de ces radicaux hétérocycliques pouvant être substitué par un ou plusieurs des radicaux suivants: halogéno, $C_1$—$C_6$-alcoxy, $C_7$—$C_{12}$-aralcoxy, hydroxyle, $C_1$—$C_6$-hydroxyalcoyle, $C_1$—$C_6$-alcoxy($C_1$—$C_6$)alcoyle, trifluorométhyle, $C_1$—$C_6$-alcoyle, phényle ou $C_7$—$C_{12}$-aralcoyle, ou disubstitué par un radical alcoylènedioxy inférieur de 1 à 6 atomes de carbone et le radical phényle ou la partie aryle d'un radical aralcoyle pouvant être substitué par l'un quelconque des substituants ci-dessus, et x représente 0 ou 1, et les sels pharmaceutiquement acceptables de celui-ci.

2. Composé suivant la revendication 1, dans lequel Het est un radical pyridyle, quinoléyle ou thiazolyle qui peut être substitué par un ou plusieurs des radicaux suivants: halogéno, $C_1$—$C_6$-alcoxy, $C_7$—$C_{12}$-aralcoxy, hydroxyle, $C_1$—$C_6$-hydroxyalcoyle, $C_1$—$C_6$-alcoxy($C_1$—$C_6$)alcoyle, trifluorométhyle, $C_1$—$C_6$-alcoyle, phényle ou $C_7$—$C_{12}$-aralcoyle, ou disubstitué par un radical alcoylènedioxy inférieur de 1 à 6 atomes de carbone et le radical phényle ou la partie aryle du radical aralcoyle peut être substitué par l'un quelconque des substituants ci-dessus.

3. Composé suivant la revendication 1 ou 2, dans lequel Het est uni à la position 2.

4. Composé suivant la revendication 1 suivant la revendication 1 de formule IA

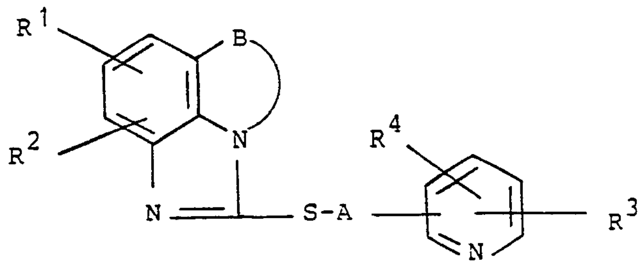

(Ia)

où

A, $R^1$ et $R^2$ sont tels que définis dans la revendication 1 à propos de la formule I,

$R^3$ et $R^4$ sont identiques ou différents et sont un atome d'hydrogène ou radical $C_1$—$C_6$-alcoyle ou phényle qui peut être substitué par l'un quelconque des substituants décrits à propos de Het dans la revendication 1 et l'un d'entre $R^3$ et $R^4$ est autre qu'un atome d'hydrogène, et les sels pharmaceutiquement acceptables de celui-ci.

5. Composé suivant l'un quelconque des revendications 1 à 4, dans lequel A est $CH_2$.

6. Composé suivant l'une quelconque des revendications précédentes, dans lequel $R^1$ et $R^2$ sont choisis entre un atome d'hydrogène et un radical $C_1$—$C_6$-alcoyle ou $C_1$—$C_6$-alcoxy.

7. Composé suivant l'une quelconque des revendications 1 à 5, dans lequel Het est un radical 2-(6-phényl-pyridyle).

8. Composé choisi parmi

la 5,6-dihydro-2-(2-pyridylméthylthio)-4H-imidazo-[4,5,1-ij]quinoléine ou un sel pharmaceutiquement acceptable de celle-ci,

la 2-(2-pyridylméthylsulfinyl)-5,6-dihydro-4H-imidazo-[4,5,1-ij]quinoléine ou un sel pharmaceutiquement acceptable de celle-ci,

la 5,6-dihydro-2-(2-(3-méthylpyridyl)méthylthio)-4-[H]-imidazo-[4,5,1-ij]quinoléine ou un sel pharmaceutiquement acceptable de celle-ci,

la 2-(2-pyridylméthylthio)-4-[H]-imidazo-[4,5,1-ij]quinoléine ou un sel pharmaceutiquement acceptable de celle-ci,

la 5,6-dihydro-2-(2-(3-méthylpyridyl)méthylsulfinyl)-[4H]-imidazo-[4,5,1-ij]quinoléine ou un sel pharmaceutiquement acceptable de celle-ci,

la 5,6-dihydro-2-(2-(6-phénylpyridyl)méthylthio)-[4H]-imidazo-[4,5,1-ij]quinoléine ou un sel pharmaceutiquement acceptable de celle-ci,

la 5,6-dihydro-2-(2-(3,5-diméthylpyridyl)méthylthio)-[4H]-imidazo-[4,5,1-ij]quinoléine ou un sel pharmaceutiquement acceptable de celle-ci,

la 5,6-dihydro-2-(2-(3,5-diméthylpyridyl)méthylsulfinyl)-4H-imidazo-[4,5,1-ij]quinoléine ou un sel pharmaceutiquement acceptable de celle-ci,

la 5,6-dihydro-2-(6-phényl-2-pyridylméthylthio)-4H-8-imidazo-[4,5,1-ij]quinoléine ou un sel pharmaceutiquement acceptable de celle-ci,

la 5,6-dihydro-2-(3-pyridylméthylthio)-4H-imidazo-[4,5,1-ij]quinoléine ou un sel pharmaceutiquement acceptable de celle-ci,

la 5,6-dihydro-2-(4-pyridylméthylthio)-4H-imidazo-[4,5,1-ij]quinoléine ou un sel pharmaceutiquement acceptable de celle-ci.

9. La 5,6-dihydro-2-(2-quinoléylméthylthio)-4H-imidazo-[4,5,1-ij]quinoléine ou un sel pharmaceutiquement acceptable de celle-ci.

La 5,6-dihydro-2-[(2-phénylthiazol-4-yl)méthylthio]-4H-imidazo-[4,5,1-ij]quinoléine ou un sel pharmaceutiquement acceptable de celle-ci.

11. Composé suivant l'une quelconque des revendications précédentes, à utiliser comme agent pharmaceutique.

12. Composé suivant l'une quelconque des revendications 1 à 10, à utiliser comme agent anti-ulcéreux ou pour le traitement de l'hypersécrétion.

13. Composition pharmaceutique comprenant un composé suivant l'une quelconque des revendications 1 à 10 et un excipient pharmaceutiquement acceptable.

14. Composition pharmaceutique suivant la revendication 13, sous form dosée unitaire.

**Revendications pour l'Etats Contactant: AT**

1. Procédé de preparation d'un composé de formule I

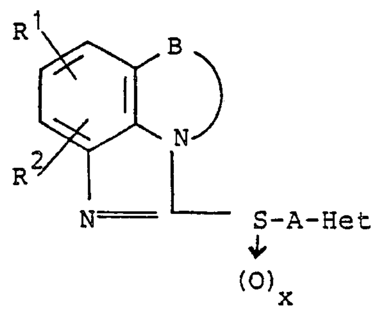

(I)

où

A est une chaîne alcoylène droite ou ramifiée en $C_1$—$C_4$ qui peut être saturée ou insaturée,

B est une chaîne alcoylène droite ou ramifiée en $C_2$—$C_4$ qui peut être saturée ou insaturée,

$R^1$ et $R^2$ sont identiques ou différents et sont un atome d'hydrogène ou radical $C_1$—$C_6$-alcoyle, $C_1$—$C_6$-alcoxy, $C_1$—$C_6$-alcoxy($C_1$—$C_6$)alcoyle, $C_1$—$C_6$-hydroxyalcoyle, hydroxyle, halogéno, nitro, carboxyle, ester carboxylique, carbamoyle, carbamoyloxy, cyano, $C_2$—$C_7$-alcanoyle, $C_2$—$C_7$-alcanoylamino ou trifluorométhyle,

Het est un radical hétérocyclique choisi parmi imidazolyle, imidazolinyle, benzimidazolyle, thiazolyle, thiazolinyle, quinoléyle, pipéridyle, pyridyle, benzothiazolyle et pyrimidyle, l'un quelconque de ces radicaux hétérocycliques pouvant être substitué par un ou plusieurs des radicaux suivants: halogéno, $C_1$—$C_6$-alcoxy, $C_7$—$C_{12}$-aralcoxy, hydroxyle, $C_1$—$C_6$-hydroxyalcoyle, $C_1$—$C_6$-alcoxy($C_1$—$C_6$)alcoyle, trifluorométhyle, $C_1$—$C_6$-alcoyle, phényle ou $C_7$—$C_{12}$-aralcoyle, ou disubstitué par un radical alcoylènedioxy inférieur de 1 à 6 atomes de carbone et le radical phényle ou la partie aryle d'un radical aralcoyle pouvant être substitué par l'un quelconque des substituants ci-dessus, et x représente 0 ou 1, et les sels pharmaceutiquement acceptables de celui-ci, caractérisé en ce que

A) un composé de formule II

Het-A-Hal (II)

où Het et A sont tels que définis ci-dessus et Hal est un atome d'halogène, est mis à réagir avec un thiol de formule III ou un sel de métal acalin de celui-ci

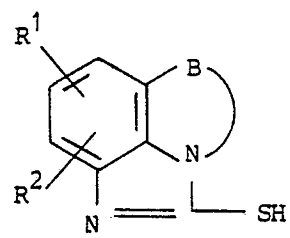

(III)

où B, $R^1$ et $R^1$ sont tels que définis ci-dessus, ou

B) un composé de formule IIa

Het-A-Sh (IIa)

ou un sel de métal alcalin de celui-ci, où Het et A sont tels que définis ci-dessus, est mis à réagir avec un halogénure de formule IIIa

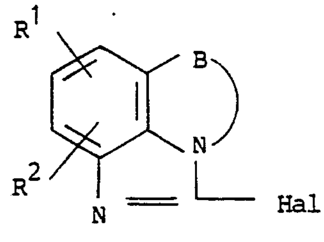

(IIIa)

où B, $R^1$, $R^2$ et Hal sont tels que définis ci-dessus, et facultativement, lorsqu'un composé de formule I où x représente 1 est souhaité, le produit est oxydé pour donner un composé de formule I où x représente 1, ou

C) un composé de formule IV

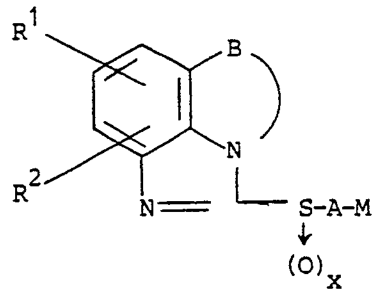

(IV)

où $R^1$, $R^2$, A, B et x sont tels que définis ci-dessus, et M représente le sodium, le potassium ou le lithium, est mis à réagir avec un composé de formule

Het-Q (V)

où Het est tel que défini ci-dessus et Q est un radical partant tel qu'un radical hydroxyle estérifié réactif ou un halogène, et si la chose est souhaitée, un produit où x représente 0 ou 1 est converti en un sel pharmaceutiquement acceptable.

2. Procédé de préparation d'un composé de formule I tel que défini dans la revendication 1 ou d'un sel pharmaceutiquement acceptable de celui-ci, ou x represente 1, caractérisé en ce qu'un composé correspondant de formule I où x représente 0 ou un sel pharmaceutiquement acceptable de celui-ci est oxydé et si la chose est souhaitée, le produit est isolé à l'état de base libre ou de sel pharmaceutiquement acceptable.

3. Procédé suivant la revendication 1, caractérisée en ce qu'on utilise un composé de départ dans lequel Het est un radical pyridyle, quinoléyle ou thiazolyle qui peut être substitué par un ou plusieurs des radicaux suivants: halogéno, $C_1$—$C_6$-alcoxy, $C_7$—$C_{12}$-aralcoxy, hydroxyle, $C_1$—$C_6$-hydroxyalcoyle, $C_1$—$C_6$-alcoxy($C_1$—$C_6$)-alcoyle, trifluorométhyle, $C_1$—$C_6$-alcoyle, phényle ou $C_7$—$C_{12}$-aralcoyle, ou disubstitué par un radical alcoylènedioxy inférieur de 1 à 6 atomes de carbone et le radical phényle ou la partie aryle du radical aralcoyle peut être substitué par l'un quelconque des substituants ci-dessus.

4. Procédé suivant la revendication 1, 2 ou 3, caractérisé en ce qu'on utilise un composé de départ dans lequel A représente $CH_2$.

5. Procédé suivant la revendication 3, caractérisé en ce qu'on utilise un composé de départ dans lequel Het représente

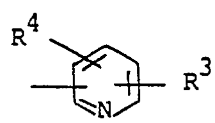

où $R^3$ et $R^4$ sont identiques ou différents et sont un atome d'hydrogène ou radical $C_1$—$C_6$-alcoyle ou phényle qui peut être substitué par l'un quelconque des substituants décrits à propos de Het dans la revendication 1 et l'un d'entre $R^3$ et $R^4$ est autre qu'un atome d'hydrogène, ou ses sels pharmaceutique acceptables.

6. Procédé suivant la revendication 5, caractérisé en ce qu'on utilise un composé de départ dans lequel Het représente un radical 2-(6-phénylpyridyle).

7. Procédé suivant la revendication 1, caractérisé en ce qu'on utilise un composé de départ dans lequel Het représente un radical 2-phénylthiazol-4-yle ou 2-quinoléyle.

8. Procédé de préparation d'une composition pharmaceutique, caractérisé en ce qu'un composé de formule I tel que défini dans la revendication 1 est préparé par un procédé suivant l'une quelconque des revendications 1 à 7 est ensuite mélangé avec un excipient pharmaceutique, puis la composition résultante est façonnée à une forme propre à l'administration thérapeutique.

9. Procédé suivant la revendication 8, caractérisé en ce que la composition est présentée à l'état de comprimés, de capsules ou d'autres formes dosées unitaires.